# EUROPÄISCHE PATENTSCHRIFT

(11) **EP 2 605 811 B1**
(45) Veröffentlichungstag und Bekanntmachung des Hinweises auf die Patenterteilung: **21.10.2015**
(21) Anmeldenummer: 11745700.2
(22) Anmeldetag: 12.08.2011
(51) Int. Cl.: A61M 1/36

(54) **VORRICHTUNG ZUR ERMITTLUNG UND/ODER ÜBERWACHUNG VON FREMDSTRUKTUREN IN EINEM FLUID ODER EINEM FLUIDSTROM SOWIE VERFAHREN HIERZU**
DEVICE FOR DETERMINING AND/OR MONITORING FOREIGN STRUCTURES IN A FLUID OR IN A FLUID STREAM, AND METHOD FOR DOING SAME
DISPOSITIF DE DÉTECTION ET/OU DE CONTRÔLE DE STRUCTURES ÉTRANGÈRES DANS UN FLUIDE OU DANS UN ÉCOULEMENT DE FLUIDE

(30) Priorität: 17.08.2010 DE 102010034553
(43) Veröffentlichungstag der Anmeldung: 26.06.2013
(73) Patentinhaber: Fresenius Medical Care Deutschland GmbH, 61352 Bad Homburg (DE)
(72) Erfinder: WIKTOR, Christoph, 63571 Gelnhausen (DE)
(74) Vertreter: Herrmann, Uwe
(86) Internationale Anmeldenummer: PCT/EP2011/004072
(87) Internationale Veröffentlichungsnummer: WO 2012/022456

(56) Entgegenhaltungen:
- EP-A2- 0 980 686
- JP-A- 7 103 967
- US-A1- 2006 287 628
- US-A1- 2009 078 047
- US-B1- 6 740 036

## Beschreibung

Die vorliegende Erfindung betrifft eine Vorrichtung zur Ermittlung und/oder Überwachung von Fremdstrukturen in einem Fluid oder einem Fluidstrom sowie insbesondere ein Verfahren hierzu.

Im extrakorporalen Blutkreislauf z. B. eines Hämodialysesystems ist es von Bedeutung, dass Blut hinsichtlich gefährlicher Bestandteile, die durch die Behandlung auftreten können, überwacht wird. Insbesondere ist streng darauf zu achten, dass Gasblasen und Blutgerinnsel im extrakorporalen Blutkreislauf erkannt und/oder zurückgehalten werden können. Bekannte Behandlungssysteme sind dabei in der Lage, auf die Erkennung von Luftblasen zu reagieren und einen entsprechenden Alarm und/oder Behandlungsstopp zu bewirken. Des Weiteren wird versucht, Blutgerinnsel mittels Gerinnselfänger zurückzuhalten.

Gängige Untersuchungen von Blutproben hinsichtlich ihrer Bestandteile oder ihrer Strömungscharakteristik können z. B. mit Ultraschallmessungen basierend auf verschiedenen Prinzipien oder durch optische Messungen erfolgen. Mit einer Ultraschallanalyse durch entsprechend ausgerichtete Frequenzen können z. B. Gasblasen im Blut erkannt werden. Das Auftreten von Blutgerinnseln kann dagegen nicht mit dem gleichen Ultraschallprinzip erfasst werden, da die Streuwirkung des Schalls an den Gerinnseln in der Regel zu gering ist. Auch kann es der Fall sein, dass der Dichteunterschied im Blutabschnitt von Gerinnseln nicht von anderen Abschnitten zu unterscheiden ist. Optische Messungen können das Auftreten von Gerinnseln durch eine Änderung der Transmission von Licht erkennen. Allerdings können Gerinnsel gleichzeitig aber nicht von Gasblasen unterschieden werden, die ebenfalls eine Transmissionszunahme hervorrufen.

Problematisch ist, dass es trotz Antikoagulation nicht ausgeschlossen ist, dass im extrakorporalen Blutkreislauf einer Dialysemaschine Blutgerinnsel entstehen. Die Gründe dafür können vielfältig sein und nicht immer ist die falsche Dosierung von Antikoagulantien wie Heparin oder Citrat die Ursache. Blutstillstand, Stagnationszonen sowie der Kontakt mit Luft oder den künstlichen Oberflächen des extrakorporalen Blutkreislaufs begünstigen eine Aktivierung der Gerinnungskaskade.

Die Thromobozyten oder Blutplättchen im Blut verändern nach Aktivierung der Gerinnung ihre Form und aggregieren zu Thromben. Diese Thromben können die Kapillaren des Dialysators verstopfen und, sofern sie sich nicht im extrakorporalen Blutkreislauf verfangen, in den Kreislauf des Patienten gelangen und dort kleinere Gefäße verschließen. Aus diesem Grund gibt es in vielen aktuellen Blutschlauch- und Kassettensystemen venöse Gerinnselfänger, die Blutgerinnsel auffangen sollen, bevor sie in den Körper eines Patienten gelangen. Diese Schutzmaßnahme ist jedoch nicht unumstritten.

Möchte man nun nach einer Dialysebehandlung das Blut nicht venös, sondern arteriell zurückgeben, so schafft ein mechanischer Gerinnselfänger auf arterieller Seite keine Abhilfe, da bei Strömungsumkehr zuvor gefangene Gerinnsel wieder freigesetzt werden und in den Patienten gelangen können.

Aus dem Stand der Technik sind bereits einige Ansätze zur Überwachung eines Fluidstroms auf darin vorkommende Gasblasen oder Festkörper bekannt.

Die US 4,122,713 offenbart beispielsweise ein Messsystem zur Ermittlung der Flussgeschwindigkeit eines Fluids. Dieses System ist dabei auch in der Lage, neben der Messung der Blutflussgeschwindigkeit auch die Anwesenheit von Luftblasen im Blut zu ermitteln.

Die EP 0 979 111 B1 betrifft eine Vorrichtung zur optischen Erkennung und Quantifizierung von Mikrobläschen im Blut. Dabei wird auch eine optische Erkennung von Blutgerinnseln beschrieben, die in dieser Form jedoch nur von Partikeln mit einer entsprechenden Größe angenommen werden kann, nicht jedoch für kleine Blutgerinnsel.

Die JP 7103967 beschreibt eine Anordnung zur Untersuchung von Blutserumproben, die sich in einem Probengefäß wie z. B. einem Messbecher befinden. Mittels optischer Sensoren und einem Ultraschallsensor wird eine Füllstandsmessung und eine Phasengrenzdetektion von in den Probengefäßen befindlichen separierten Blutproben vorgenommen.

Aus der WO 2007/121398 A2 ist weiter ein System zur Detektion von Gasblasen und festen Partikeln in Blut durch Radiowellenanalyse bekannt.

Die US 3,935,876 offenbart eine Vorrichtung zur Überwachung eines Blutstroms hinsichtlich Luftblasen mit optischen Mitteln, wobei zugleich mit der Vorrichtung eine Überwachung auf Blutgerinnsel stattfinden kann.

Die US 2009/078047 A1 offenbart ein nicht-invasives, multifunktionales Sensorsystem mit einer Aussparung für einen Schlauch. In der Wandung der Aussparung sind mehrere Sensoren angeordnet, die beispielsweise optisch oder mittels Ultraschall verschiedene Parameter einer Flüssigkeit und u.a. auch das Vorhandensein von Luftblasen oder Gerinnseln messen können.

Die EP 0 980 686 A2 beschreibt einen Multifunktionssensor zur Lufterkennung und Drucküberwachung sowie zur Messung von Blutvolumen und Bluttemperatur mittels optischen und eines ultraschallbasierten Verfahren.

Die US 6 740 036 B1 betrifft eine Vorrichtung zur Messung mehrerer Parameter in einer medizinischen Flüssigkeit, die durch eine Messkammer geführt wird. Die Messkammer besitzt eine flexible Membran, auf der eine Messplatte mit mindestens einem Sensor aufliegt.

Aus der US 2006/0287628 A1 ist eine Vorrichtung zur Gewinnung von Blutkomponenten bekannt, die mehrere Sensoren zur Trübungs-, Gasblasen- und Gewichtsmessung enthält.

Trotz der bisherigen Ansätze wäre es wünschenswert, das Auftreten von Blutgerinnseln, insbesondere auch kleinen Blutgerinnseln sowie Gasblasen im Blut nicht nur schnell und sicher zu erkennen sondern auch beides voneinander zu unterscheiden, wobei eine entsprechende Messeinrichtung möglichst einfach aufgebaut ist.

Es ist daher die Aufgabe der vorliegenden Erfindung, eine Vorrichtung und ein Verfahren der eingangs genannten Art in vorteilhafter Weise weiterzubilden, insbesondere dahingehend, dass ein einfacheres und sichereres Verfahren sowie eine einfach und sicher betreibbare Vorrichtung bereitgestellt werden, wobei vorteilhafterweise eine sichere und hochgenaue Überwachung eines Blutstroms hinsichtlich Luftblasen und Blutgerinnsel möglich ist.

Diese Aufgabe wird erfindungsgemäß gelöst durch eine Vorrichtung mit den Merkmalen des Anspruchs 1. Danach ist vorgesehen, dass eine Vorrichtung zur Ermittlung und/oder Überwachung von Fremdstrukturen in einem Fluid oder einem Fluidstrom mit wenigstens einem optischen Überwachungsmittel, wenigstens einem Ultraschallüberwachungsmittel und wenigstens einem Signalauswertemittel versehen ist, wobei das Fluid zumindest mittels des optischen Überwachungsmittels optisch und zumindest mittels des Ultraschallüberwachungsmittels mittels Ultraschall überwachbar ist und wobei anhand der Kombination der hieraus erhaltenen Überwachungssignale wenigstens eine Fremdstruktur, insbesondere eine Luftblase und/oder ein Festkörper wie ein Blutgerinnsel, im Fluid mittels des Signalauswertemittels erkennbar und/oder von wenigstens einer zweiten Fremdstruktur mittels des Signalauswertemittels unterscheidbar ist.

Das Fluid kann insbesondere Blut sein und der Fluidstrom kann insbesondere ein Blutstrom sein.

Die Kombination der Überwachungssignale zur Erkennung der wenigstens einen Fremdstruktur bzw. zur Unterscheidung der ersten Fremdstruktur von wenigstens einer zweiten Fremdstruktur kann vorzugsweise anhand des Abgleichs der Überwachungssignale des optischen Überwachungsmittels und des Ultraschallüberwachungsmittels erfolgen.

Die Fremdstruktur kann beispielsweise eine Luftblase und/oder ein Festkörper wie ein Blutgerinnsel sein, insbesondere stellt eine Luftblase eine erste Fremdstruktur und ein Festkörper wie ein Blutgerinnsel eine zweite Fremdstruktur dar, die mittels der Vorrichtung vorteilhafterweise erkennbar und unterscheidbar sind.

Wird mittels des Ultraschallüberwachungsmittels eine Fremdstruktur detektiert, so wird hierdurch eine Luftblase erkannt und dies grundsätzlich auch unabhängig davon, ob dies mittels des optischen Überwachungsmittels ebenfalls detektiert wird. Im Normalfall und vorzugsweise wird eine Luftblase sowohl durch das Ultraschallüberwachungsmittel als auch durch das optische Überwachungsmittel detektiert. Dies ist jedoch zur Detektion von Luftblasen nicht zwingend notwendig.

Dadurch ergibt sich der Vorteil, einfach und zuverlässig arbeitende Überwachungsmethoden einsetzen zu können, nämlich ein optisches Überwachungsmittel und ein Ultraschallüberwachungsmittel. Bei dem optischen Überwachungsmittel kann beispielsweise das Messprinzip einer einfachen Transmissionsmessung genutzt werden. Bei der Ultraschallüberwachung sind grundsätzlich alle Messprinzipien möglich, durch die z. B. Gasblasen detektiert werden können.

Beispielsweise kann das Ultraschallüberwachungsmittel an einer Seite eines Fluidführungsweges mit seinem Empfängerteil angeordnet sein. Der Ultraschallimpuls kann sodann von einem Sender in die mit Flüssigkeit durchströmte Fluidführung eingekoppelt werden. Die Gasblasen im Fluid schwächen das akustische Signal ab. Nach Durchwandern des Schallsignals durch die Fluidführung trifft das Signal auf die Wandung des Fluidführungskanals und wird entsprechend reflektiert. Dieses Signal durchwandert sodann erneut die Fluidführung und kann vom Empfängerteil aufgenommen werden. Anhand der Auswertung des Schallsignals kann sodann unterschieden werden, ob Gasblasen vorhanden sind oder nicht.

Durch die kombinierte Auswertung der Überwachungssignale, die vom optischen Überwachungsmittel und vom Ultraschallüberwachungsmittel erhalten werden können, ist es vorteilhaft möglich, einfach und sicher das Vorhandensein einer Luftblase und/oder eines Festkörpers zu erkennen.

Ein entsprechendes Signalauswertemittel kann beispielsweise eine Recheneinheit sein, wobei es sich auch um die Recheneinheit eines mit der Vorrichtung in Verbindung stehenden Steuerungs- und/oder Regelungsmittels einer Blutbehandlungsvorrichtung wie einer Hämodialysemaschine handeln kann.

Des Weiteren kann vorgesehen sein, dass mittels des Signalauswertemittels eine oder mehrere Luftblasen im Fluid erkennbar sind, wenn zumindest das Ultraschallüberwachungsmittel wenigstens eine Fremdstruktur als ein signalauslösendes Ereignis registriert und ein Signal abgibt oder wenn das optische Überwachungsmittel und das Ultraschallüberwachungsmittel jeweils wenigstens eine Fremdstruktur als ein signalauslösendes Ereignis im Wesentlichen zeitgleich registrieren und ein Signal abgeben, und/oder dass mittels des Signalauswertemittels ein oder mehrere Festkörper, insbesondere Blutgerinnsel, im Fluid erkennbar sind, wenn das optische Überwachungsmittel wenigstens eine Fremdstruktur als signalauslösendes Ereignis registriert und das Ultraschallüberwachungsmittel im Wesentlichen zeitgleich keine oder eine sehr kleine Fremdstruktur als signalauslösendes Ereignis registriert. Eine zeitgleiche Messung kann verständlicherweise nur erfolgen, wenn das optische Überwachungsmittel und das Ultraschallüberwachungsmittel am gleichen Ort angeordnet sind bzw. am gleichen Ort signalauslösende Ereignisse registrieren. Wenn das optische Überwachungsmittel und das Ultraschallüberwachungsmittel nicht am gleichen Ort, sondern versetzt angeordnet sind bzw. an unterschiedlichen Orten signalauslösende Ereignisse registrieren, so ist dieser Versatz z. B. durch Berücksichtigung der Strömungsgeschwindigkeit und der damit verbundenen Totzeit zu berücksichtigen. Auch in einem derartigen Fall erfolgt die Registrierung der signalauslösenden Ereignisse durch die Berücksichtigung des Versatzes dann ebenfalls im Wesentlichen zeitgleich im vorgenannten Sinne. Insbesondere sind optische Überwachungsmittel und das Ultraschallüberwachungsmittel vorzugsweise nur mit einem geringen bzw. sehr geringen Versatz angeordnet. Es ist in diesem Zusammenhang auch denkbar, dass ein Gerinnsel dann erkannt werden kann, wenn das optische Signal wesentlich größer bzw. stärker ist als das erhaltene Ultraschallsignal. Anhand der Divergenz der erhaltenen Signale kann somit ebenfalls ein Gerinnsel detektiert werden.

Es ist möglich, dass das optische Überwachungsmittel wenigstens ein optischer Sensor ist oder wenigstens einen optischen Sensor umfasst und/oder dass das Ultraschallüberwachungsmittel wenigstens ein Ultraschallsensor ist oder wenigstens einen Ultraschallsensor umfasst und/oder dass mittels des optischen Überwachungsmittels Luftblasen und Festkörper, insbesondere Blutgerinnsel, detektierbar und mittels des Ultraschallüberwachungsmittels Luftblasen detektierbar sind. Der optische Sensor kann auf Luftblasen und Blutgerinnsel gleichermaßen mit einem kurzen und starken Signalanstieg reagieren, da die transmittierte Intensität der Strahlung kurzzeitig zunimmt. Demgegenüber kann ein Ultraschallsensor nur vorbeiströmende Luftblasen detektieren und quantifizieren. Eine Gerinnselerkennung kann durch den Ultraschallsensor nicht erfolgen, da der Dichteunterschied zwischen Blut bzw. Plasma und Gerinnsel zu gering ist. Folglich kann in einem Fall, in dem nur der optische Sensor ein Signal generiert und der Ultraschallsensor kein Signal generiert, festgestellt werden, dass sich ein Blutgerinnsel im Blutstrom befindet. Entsprechend kann bzw. können eine oder mehrere Luftblasen detektiert werden, wenn beide Sensoren, also der optische Sensor und der Ultraschallsensor ein Signal generieren, festgestellt werden.

Vorzugsweise kann vorgesehen sein, dass der optische Sensor wenigstens eine Lichtquelle und wenigstens einen Photodetektor aufweist, wobei die Lichtquelle vorzugsweise wenigstens eine LED ist oder umfasst und/oder wobei mittels der Lichtquelle besonders vorteilhaft schmalbandiges, nahinfrarotes Licht mit einer Spitzenwellenlänge von etwa 805 nm ausstrahlbar ist. Der Wellenlängenbereich um 805nm eignet sich für den optischen Sensor zur Gerinnseldetektion insbesondere deswegen gut, da die Absorption durch das Hämoglobin in den Erythrozyten einerseits sehr gering und andererseits unabhängig vom Sauerstoffgehalt ist. Die Signalgenerierung ist folglich vorteilhafterweise von entsprechenden Störfaktoren und Signalverfälschungen entsprechend befreit.

Thrombozyten besitzen bei 805 nm einen sehr niedrigen Absorptionskoeffizienten (nicht messbar), jedoch findet an den Zellen eine Streuung statt, die (wie bei Erythrozyten) im Wesentlichen vorwärts gerichtet ist. Im Normalfall (ohne Thrombenbildung) ist diese Streuung jedoch vernachlässigbar, da die Anzahl und Größe der Thrombozyten im Vergleich zu Erythrozyten sehr gering ist: 99% der Blutzellen sind Erythrozyten mit einem Durchmesser von ca. 7,5 µm (Thrombozyten haben einen Durchmesser von 1,5-3 µm). Beim Einsetzen der Gerinnung aggregieren Thrombozyten, bilden einen Thrombus und nehmen mehr Volumen ein. Das führt zu verringerter Absorption innerhalb der Messstrecke und damit zu einem Anstieg der transmittierten Strahlung bei 805 nm.

Darüber hinaus ist denkbar, dass signalabwärts des optischen Überwachungsmittels wenigstens ein Hochpassfilter angeordnet ist, mittels dessen das mittels optischer Überwachung gewonnene Überwachungssignal filterbar ist. Vorteilhafterweise kann es sich bei dem Hochpassfilter um einen Filter handeln, in dem das Überwachungssignal in festen Abständen von wenigen Millisekunden integrierbar und aus einer vorbestimmten Anzahl von vorangegangener Messungen ein gleitender Mittelwert bestimmbar und vom aktuellen Integrationsergebnis subtrahierbar ist. Ein derartiger Filter weist den Vorteil auf, dass er eine einfache Auswertung des Signals ermöglicht. Wird bei einem solchen Filter das Integrationsergebnis höher gewichtet als der Tiefpass, so erhält man einen High-Boost-Filter, der den Vorteil hat, dass der prinzipielle Signalverlauf erhalten bleibt, während die kurzzeitigen Änderungen hervorgehoben werden. Überschreitet das hochpassgefilterte Signal einen Schwellwert, so ist entweder eine Luftblase oder ein Gerinnsel durch die Messstrecke geströmt. Detektiert der Ultraschallsensor zeitnah unter Berücksichtigung der Strömungsgeschwindigkeit ebenfalls ein eigenes Signal, so handelt es sich um eine Luftblase. Registriert der Ultraschallsensor jedoch nichts, so wurde ein Gerinnsel detektiert.

Grundsätzlich sind jedoch auch andere Hochpassfilter zur Auswertung geeignet.

Außerdem ist möglich, dass die Vorrichtung eine Aufnahme aufweist, in die ein Fluidführungsmittel, vorzugsweise ein Schlauchset oder ein Teil eines Schlauchsets oder eine Kassette, insbesondere eine Disposablekassette, oder ein Messkanal, einsetzbar ist und/oder dass die Vorrichtung einen Messkanal aufweist. Bei einem derartigen Schlauchset kann es sich insbesondere um das Schlauchset eines extrakorporalen Blutkreislaufs für eine Hämodialysemaschine handeln. Des Weiteren kann es sich bei einer derartigen Disposablekassette um eine Disposablekassette handeln, in der Teile eines extrakorporalen Blutkreislaufs für eine Hämodialysemaschine angeordnet sind.

Ferner ist denkbar, dass die Vorrichtung eine begrenzte Messstrecke oder Messstelle aufweist oder ausbildet, an der sowohl das optische Überwachungsmittel als auch das Ultraschallüberwachungsmittel angeordnet sind. Durch die örtliche Nähe des optischen Überwachungsmittels zum Ultraschallüberwachungsmittel kann die Überwachung auf Luftblasen bzw. Blutgerinnsel vorteilhaft verbessert werden.

Besonders vorteilhaft ist es, wenn die Messstrecke oder Messstelle in und/oder an der Aufnahme angeordnet ist und/oder dass die Messstrecke oder Messstelle von der Lichtquelle, dem Ultraschallsensor und dem Photodetektor des optischen Überwachungsmittels von drei Seiten umschlossen ist, vorzugsweise in dieser Reihenfolge und vorteilhafterweise derart angeordnet, so dass die Lichtquelle, der Ultraschallsensor und der Fotodetektor des optischen Überwachungsmittels die Messstrecke oder Messstelle U-förmig umschließen. Insbesondere kann durch die U-förmige Anordnung von Lichtquelle, Ultraschallsensor und Photodetektor eine sehr kurze Messstrecke realisiert werden mit dem Vorteil, dass eine vergleichsweise hochgenaue Überwachung der Messstrecke möglich ist. Der Zeitversatz zwischen den jeweils generierten Signalen des optischen Überwachungsmittels als auch des Ultraschallüberwachungsmittels wird hierdurch so gering wie möglich gehalten.

Grundsätzlich ist auch denkbar, dass das optische Überwachungsmittel und das Ultraschallüberwachungsmittel in Transmission und jeweils quer zur Flussrichtung angeordnet sind. Weiter ist denkbar, dass das optische Überwachungsmittel in Transmission quer zur Flussrichtung und das Ultraschallüberwachungsmittel längs zur Flussrichtung angeordnet sind.

Des Weiteren kann vorgesehen sein, dass die Vorrichtung wenigstens ein Schutzmittel und/oder wenigstens ein Warnmittel aufweist und/oder mit wenigstens einem Schutzmittel und/oder wenigstens einem Warnmittel in Verbindung steht, wobei mittels des Schutzmittels vorzugsweise der Fluidstrom stoppbar ist und/oder wobei vorzugsweise mittels des Warnmittels auf im Fluid erkannte Bestandteile, insbesondere Luftblasen und Festkörper wie Blutgerinnsel hinweisbar ist. Das Warnmittel kann beispielsweise ein akustisches und/oder optisches Warnmittel sein. Insbesondere kann vorgesehen sein, dass mittels des Warnmittels etwa auf einem Bildschirm ein Warnhinweis ausgegeben wird, wobei zugleich ein Warnton ertönt. Das Schutzmittel kann beispielsweise als Klemme ausgeführt sein oder eine Klemme umfassen und zugleich vorzugsweise mit einem Antriebsmittel wie der Pumpe des Fluidstroms zusammenwirken. Dadurch kann vorteilhafterweise sichergestellt werden, dass bei Erkennung von Fremdstrukturen im Fluid sofort der Fluidstrom gestoppt und eine entsprechende Warnung ausgegeben werden kann. Dies ist insbesondere bei der Hämodialyse sehr wichtig und von Vorteil, da hierdurch eine Zuführung von Luftblasen oder Blutgerinnseln zum Patienten sicher unterbunden werden kann.

Das Schutzmittel und auch das Warnmittel können vorteilhafterweise Bestandteile einer Blutbehandlungsvorrichtung wie einer Hämodialysemaschine sein, die bei der Blutbehandlungsvorrichtung als übliche Komponenten ohnehin grundsätzlich vorhanden sind. Es ergibt sich somit der Vorteil, auf bereits bestehende Komponenten zugreifen zu können, so dass bestehende Blutbehandlungsvorrichtungen einfach nachgerüstet werden können.

Darüber hinaus kann vorgesehen sein, dass die Vorrichtung Bestandteil einer Blutbehandlungsvorrichtung, insbesondere einer Hämodialysemaschine ist oder das die Vorrichtung eine Blutbehandlungsvorrichtung, insbesondere eine Hämodialysemaschine ist.

Des Weiteren betrifft die Erfindung ein Verfahren zur Ermittlung und/oder Überwachung von Fremdstrukturen in einem Fluid oder einem Fluidstrom mit den Merkmalen des Anspruchs 10. Danach ist vorgesehen, dass zur Ermittlung und/oder Überwachung von Fremdstrukturen in einem Fluid oder einem Fluidstrom, insbesondere Blut oder Blutstrom, das Fluid optisch und mittels Ultraschall überwacht wird und wobei anhand der Kombination der hieraus erhaltenen Überwachungssignale, insbesondere anhand des Abgleichs der hieraus erhaltenen Überwachungssignale, wenigstens eine Fremdstruktur, insbesondere eine Luftblase und/oder ein Festkörper wie ein Blutgerinnsel, im Fluid erkennbar und/oder von wenigstens einer zweiten Fremdstruktur unterscheidbar ist. Insbesondere ist von Vorteil, dass eine erste Fremdstruktur wie eine Luftblase von einer zweiten Fremdstruktur wie einem Blutgerinnsel durch das erfindungsgemäße Verfahren einfach und sicher sowie zuverlässig unterscheidbar ist.

Ferner kann vorgesehen sein, dass eine oder mehrere Luftblasen im Fluid erkannt werden, wenn zumindest mittels Ultraschallüberwachung wenigstens eine Fremdstruktur im Fluid erkannt wird oder wenn sowohl mittels optischer Überwachung und Ultraschallüberwachung im Wesentlichen zeitgleich wenigstens eine Fremdstruktur im Fluid erkannt wird, und/oder dass ein oder mehrere Festkörper, insbesondere Blutgerinnsel, im Fluid erkannt werden, wenn optisch wenigstens eine Fremdstruktur im Fluid erkannt wird und mittels Ultraschall keine oder eine sehr kleine Fremdstruktur im Fluid erkannt wird. Eine zeitgleiche Messung kann verständlicherweise nur erfolgen, wenn das optische Überwachungsmittel und das Ultraschallüberwachungsmittel am gleichen Ort angeordnet sind bzw. am gleichen Ort signalauslösende Ereignisse registrieren. Wenn das optische Überwachungsmittel und das Ultraschallüberwachungsmittel nicht am gleichen Ort, sondern versetzt angeordnet sind bzw. an unterschiedlichen Orten signalauslösende Ereignisse registrieren, so ist dieser Versatz z. B. durch Berücksichtigung der Strömungsgeschwindigkeit und der damit verbundenen Totzeit zu berücksichtigen. Auch in einem derartigen Fall erfolgt die Registrierung der signalauslösenden Ereignisse durch die Berücksichtigung des Versatzes dann ebenfalls im Wesentlichen zeitgleich im vorgenannten Sinne. Insbesondere sind optische Überwachungsmittel und das Ultraschallüberwachungsmittel vorzugsweise nur mit einem geringen bzw. sehr geringen Versatz angeordnet. Es ist in diesem Zusammenhang auch denkbar, dass ein Gerinnsel dann erkannt werden kann, wenn das optische Signal wesentlich größer bzw. stärker ist als das erhaltene Ultraschallsignal. Anhand der Divergenz der erhaltenen Signale kann somit ebenfalls ein Gerinnsel detektiert werden.

Des Weiteren ist vorteilhaft möglich, dass das mittels optischer Überwachung gewonnene Überwachungssignal mittels eines Hochpassfilter gefiltert wird.

Grundsätzlich ist hierzu jegliche Art von Hochpassfiltern geeignet.

Besonders vorteilhaft ist es jedoch, wenn der Hochpassfilter vorzugsweise ein Filter ist, in dem das Überwachungssignal in festen Abständen von wenigen Millisekunden integriert und aus einer vorbestimmten Anzahl von vorangegangenen Messungen ein gleitender Mittelwert bestimmt und vom aktuellen Integrationsergebnis subtrahiert wird.

Es kann vorgesehen sein, dass das Verfahren mit wenigstens einer Vorrichtung nach einem der Ansprüche 1 bis 9 durchgeführt wird.

Ferner betrifft die vorliegende Erfindung die Verwendung einer Vorrichtung gemäß Anspruch 1 bis 9 zur Durchführung des Verfahrens gemäß Anspruch 10 bis 14 und/oder die Verwendung einer Vorrichtung gemäß Anspruch 1 bis 9 in einer Blutbehandlungsvorrichtung, insbesondere in einer Hämodialysemaschine.

Darüber hinaus betrifft die vorliegende Erfindung eine Blutbehandlungsvorrichtung, insbesondere eine Hämodialysemaschine mit den Merkmalen des Anspruchs 16. Danach ist vorgesehen, dass eine Blutbehandlungsvorrichtung, insbesondere eine Hämodialysemaschine mit wenigstens einer Vorrichtung nach einem der Ansprüche 1 bis 9 versehen ist.

Ferner betrifft die vorliegende Erfindung die Verwendung einer Blutbehandlungsvorrichtung mit den Merkmalen des Anspruchs 15. Danach ist vorgesehen, dass eine Blutbehandlungsvorrichtung, insbesondere Blutbehandlungsvorrichtung nach Anspruch 16, zur Durchführung des Verfahrens mit den Merkmalen der Ansprüche 10 bis 14 und/oder in einer Blutbehandlungsvorrichtung, insbesondere einer Hämodialysemaschine verwendet wird.

Darüber hinaus betrifft die Erfindung die Kombination eines Disposables mit einer Vorrichtung nach Anspruch 1 Danach ist vorgesehen, dass ein Disposable, insbesondere ein disposables Schlauchset oder eine Diposablekassette, zur Verwendung in einer Vorrichtung nach einem der Ansprüche 1 bis 9, vorzugsweise zum Einsatz in die Aufnahme der Vorrichtung gemäß Anspruch 5 bis 9 eingesetzt wird. Weitere Einzelheiten und Vorteile der Erfindung sollen nachfolgend in einem in der Zeichnung dargestellten Ausführungsbeispiel der Erfindung näher erläutert werden.

Es zeigen:
- Figur 1:: eine schematische Darstellung der Detektion von Luftblasen mittels Ultraschall;
- Figur 2:: eine schematische Darstellung einer erfindungsgemäßen Vorrichtung zur Ermittlung und Überwachung von Luftblasen und Blutgerinnseln in einem Blutstrom;
- Figur 3:: ein Diagramm betreffend Erfassung von Blutgerinnseln mittels des optischen Sensors; und
- Figur 4:: ein Flussdiagramm betreffend die Signalauswertung des Verfahrens und der Vorrichtung gemäß der Erfindung.

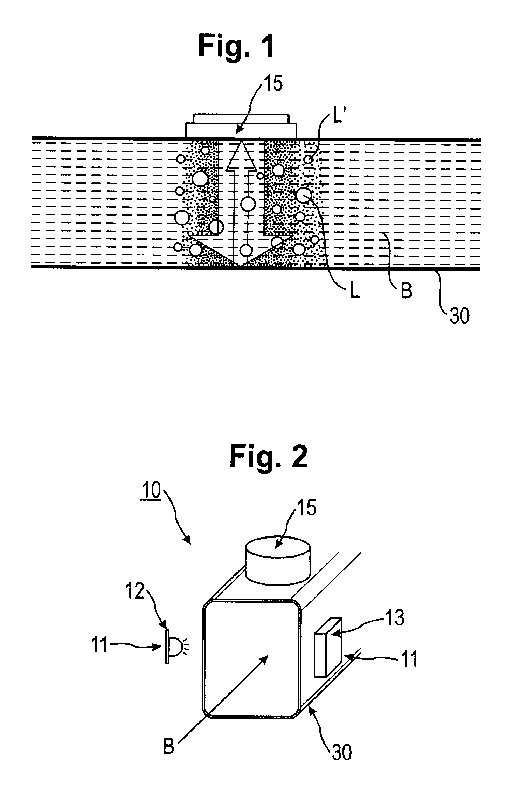

Figur 1 zeigt in schematischer Darstellung die Detektion von Luftblasen mittels Ultraschall mittels eines Ultraschallsensors 15, wie er in der Vorrichtung 10 zur Ermittlung und Überwachung von Luftblasen und Blutgerinnseln verwendet wird.

Wie in Figur 1 gezeigt, wird der Schallimpuls vom Sender des Ultraschallsensors 15 in die mit Flüssigkeit durchströmte Fluidführung 30, die ein Schlauchstück 30 eines nicht näher dargestellten disposablen Schlauchsets für die Hämodialyse sein kann, eingekoppelt. Im Blut B befindliche Gasblasen L, L' mit unterschiedlichen Größen schwächen dieses Signal ab. Nach Durchwandern des Schallsignal trifft das Signal auf die dem Sender gegenüberliegende Wand der Fluidführung 30 und wird reflektiert. Das reflektierte Signal durchwandert nun ein zweites Mal die Fluidführung 30 und wird von einem Empfänger des Ultraschallsensors 15 aufgenommen. Anhand der Auswertung des Schallsignals ist es nun grundsätzlich möglich zu unterscheiden, ob Gasblasen vorhanden sind oder nicht.

Der Ultraschallsensor 15 vereint Sender und Empfänger vorzugsweise in einem gemeinsamen Piezoelement, mittels dessen das Ultraschallsignal abgebbar und aufnehmbar ist. In einer derartigen Ausführung wird beispielsweise periodisch zwischen Senden und Empfangen umgeschaltet.

Der optische Sensor 11 zur Gerinnseldetektion (Gerinnseldetektor 11) der Vorrichtung 10 besteht, wie in Figur 2 ersichtlich, im Wesentlichen aus einer LED 12 als Lichtquelle 12, die schmalbandiges, nahinfrarotes Licht mit einer Spitzenwellenlänge von etwa 805 nm ausstrahlt. Der Wellenlängenbereich um 805 nm eignet sich deswegen besonders gut, da die Absorption durch das Hämoglobin in den Erythrozyten (rote Blutkörperchen) einerseits sehr gering und andererseits unabhängig vom Sauerstoffgehalt ist.

Gegenüber dieser Lichtquelle 12 ist zur Transmissionsmessung ein Photodetektor 13 angeordnet. Vorzugsweise ist dies ein Photodetektor 13, der ein der empfangenen Intensität entsprechendes Frequenzsignal ausgibt. Alternativ ist ein Photodetektor, der eine der Intensität proportionale Spannung oder einen der Intensität proportionalen Strom ausgibt, jedoch ebenfalls möglich.

Zwischen Lichtquelle 12 (Sender) und Photodetektor 13 (Empfänger) befindet sich die Messstrecke. Diese Messstrecke kann entweder ein eingespannter Schlauch 30 oder ein Kanal eines Kassettensystems sein. Durch diese Messstrecke strömt das Blut B. In unmittelbarer Nähe des Gerinnseldetektors 11 befindet sich der Luftblasendetektor 15 auf Ultraschallbasis, der idealerweise nur einseitig angebracht wird (vgl. auch Figur 1), so dass die Messstrecke U-förmig umschlossen wird. Auf der gegenüberliegenden Seite des Luftblasendetektors 15 befindet sich die nicht näher dargestellte Maschinenfront einer Blutbehandlungsvorrichtung, wie z. B. einer Hämodialysemaschine.

Der Ultraschallsensor 15 detektiert und quantifiziert vorbeiströmende Luftblasen. Gerinnsel kann dieser Sensor 15 nicht detektieren, da der Dichteunterschied zwischen Blut bzw. Plasma und Gerinnsel zu gering ist. Der Gerinnseldetektor 11 reagiert auf Luftblasen und Blutgerinnsel gleichermaßen mit einem kurzen, starken Signalanstieg, da die transmittierende Intensität der Strahlung kurzzeitig zunimmt. Zur Auswertung wird das Signal des Photodetektors 13 durch eine nicht näher dargestellte Auswerteeinheit in festen Abständen von einigen ms integriert. Das geschieht beim Detektor mit Frequenzausgang durch Zählen der Pulse oder Messen der Frequenz. Aus einer bestimmten Anzahl vorangegangener Messungen wird ein gleitender Mittelwert bestimmt und vom aktuellen Integrationsergebnis subtrahiert. Diese Art des Hochpassfilters ermöglicht eine einfache Auswertung des Signals. Andere Hochpassfilter eignen sich jedoch ebenfalls zur Auswertung. Überschreitet das hochpassgefilterte Signal einen Schwellwert, so ist entweder eine Luftblase oder ein Gerinnsel durch die Messstrecke geströmt. Detektiert der Ultraschallsensor 15 (zeitnah unter Berücksichtigung der Strömungsgeschwindigkeit) ebenfalls ein Ereignis, so handelt es sich um eine Luftblase. Registriert der Ultraschallsensor 15 jedoch nichts, so wurde ein Gerinnsel detektiert.

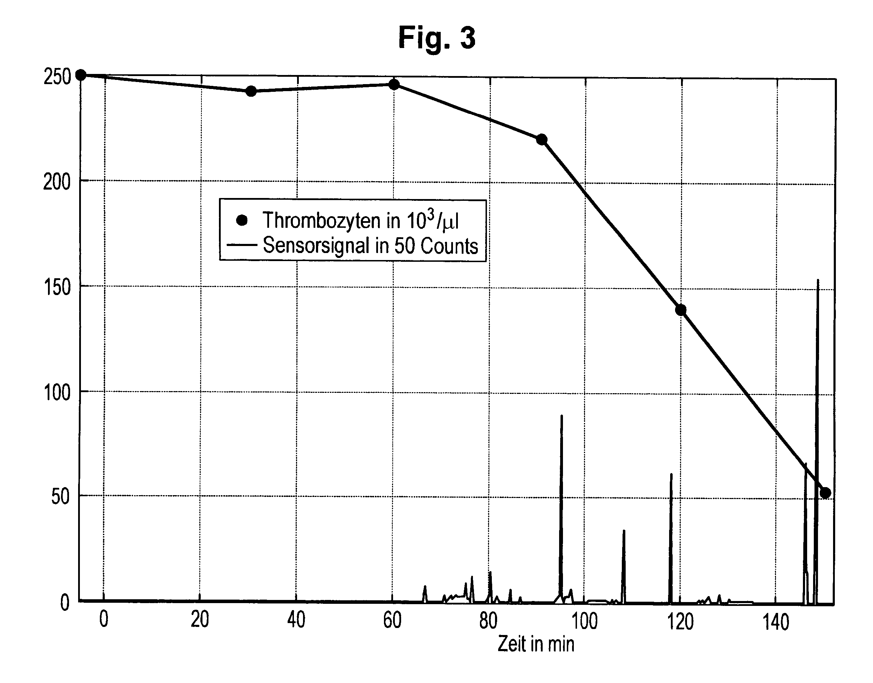

Figur 3 zeigt beispielhaft das Ergebnis einer Messung mit Blut und mit dem optischen Sensor 11. Die Gerinnung des Blutes tritt nach etwas mehr als 60 Minuten ein, was an dem Rückgang der Thrombozyten im Blut erkennbar ist. Folglich kommt es zum Auftreten von mehreren Blutgerinnsel, die mittels des Sensors 11 detektiert werden. Die Ausschläge der Sensorsignale sind als senkrechte Balken im Diagramm angetragen.

Die Höhe der Impulse kann durch Auslegung des Sensors 11 beeinflusst werden. Im hier vorliegenden Fall wurde eine Integration des Sensorsignals über 20 ms vorgenommen. Wird diese Integrationszeit kürzer gewählt, so heben sich die Impulse deutlicher vom restlichen Transmissionssignal ab. Die Auslegung des Filters bzw. Hochpassfilters - insbesondere die Anzahl der Messwerte, die in die Berechnung des gleitenden Durchschnitts einfließen - hat ebenfalls Einfluss hierauf.

Wird nun ein Gerinnsel oder eine Luftblase detektiert, so wird durch die Blutbehandlungsvorrichtung, insbesondere die Hämodialysemaschine ein Alarm ausgegeben, etwa durch einen Warnton sowie durch eine entsprechende Anzeige mittels Warnblinklicht oder auf dem Bedienbildschirm.

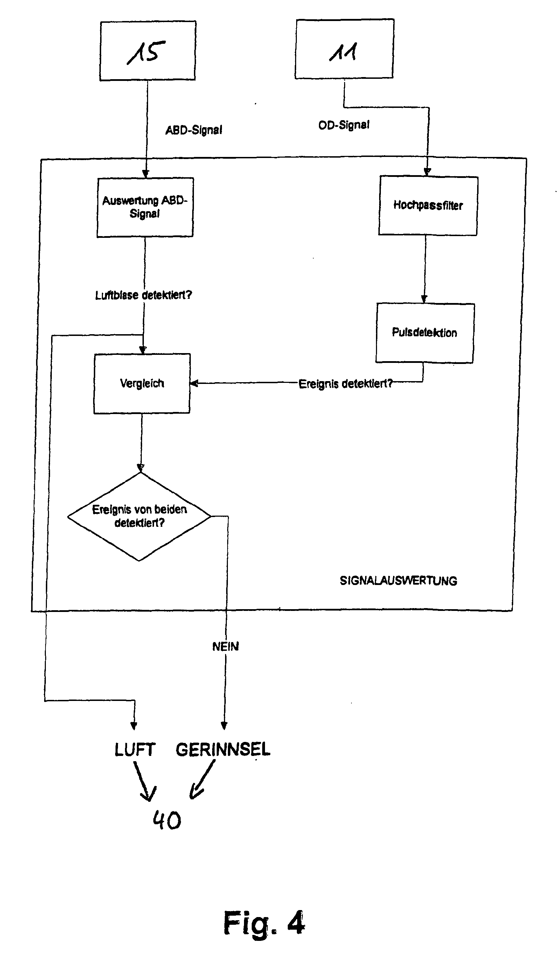

Besonders vorteilhaft kann die erfindungsgemäße Vorrichtung bei der Reinfusion von Blut aus dem extrakorporalen Blutkreislauf eingesetzt werden, denn hierbei wird Blut nicht zwangsweise durch den im venösen Teil üblicherweise vorhandenen Gerinnselfänger durchgefördert. Beispielsweise kann bei der Reinfusion im Alarmfall ein Beenden der Reinfusion oder ein Einleiten eines Sonderhandlings durch den Bediener ausgelöst werden, wobei das Sonderhandling darin bestehen kann, dass der entsprechende Fluidstrom mit Gerinnsel oder Luftblasen in einen Kochsalzbeutel oder ein sonstiges Auffangbehältnis gefördert wird. Zugleich kann aber auch für Gerinnsel oder Luftblasen, die bei normaler Flussrichtung, also durch den Luft- und Gerinnselfänger im venösen Teil des extrakorporalen Blutkreislaufs gefördert werden, keine Warnung ausgegeben werden, denn diese stellen keine Gefahr für den Patienten dar. Diese Funktionen können vorzugsweise automatisch oder halbautomatisch durch ein Schutz- und Warnmittel 40 (vgl. Figur 4) durchgeführt werden.

Denkbar ist beispielsweise, dass jeweils ein optischer Sensor 11 und ein Ultraschallsensor 15 sowohl auf der arteriellen als auch auf der venösen Seite des Blutkreislaufs vorgesehen ist. Im normalen Behandlungsbetrieb kann das Sensorpaar bestehend aus dem optischen Sensor 11 und dem Ultraschallsensor 15 auf der venösen Seite des extrakorporalen Blutkreislaufs auf Gerinnsel ausgewertet werden und bei einer Detektion ein Sonderhandling anfordern. Bei normaler Reinfusion, beispielsweise mittels eines Einförderns von Kochsalzlösung aus einem an die arterielle Leitung angeschlossenen Kochsalzlösungsbeutels und einer Reinfusion des Blutes über den venösen Teil des extrakorporalen Blutkreislaufs kann bzw. wird ebenfalls das Sensorpaar bestehend aus dem optischen Sensor 11 und dem Ultraschallsensor 15 auf der venösen Seite des extrakorporalen Blutkreislaufs ausgewertet. Bei simultaner Reinfusion, wenn also das Blut sowohl über den arteriellen und venösen Teil des extrakorporalen Blutkreislaufs zurückgegeben wird, werden beide Sensorpaare, also sowohl das Sensorpaar auf der arteriellen als auch auf der venösen Seite ausgewertet, wobei beide bei Detektion eines Gerinnsels oder einer Luftblase die Reinfusion auf der entsprechenden Seite entweder beenden oder ein Sonderhandling anfordern. In jedem Fall wird die Sicherheitsklemme genutzt, um den Blutfluss zu stoppen.

Figur 4 zeigt vereinfacht nochmals das Flussdiagramm betreffend die Signalauswertung der Vorrichtung bzw. des Verfahrens. Das vom optischen Sensor 11 aufgenommene Signal wird durch einen Hochpassfilter geleitet und einer Pulsdetektion unterzogen. Sofern ein Ereignis detektiert wurde, wird dieses mit dem vom Ultraschallsensor 15 gewonnenen Signal verglichen. Sollte im Wesentlichen zeitgleich sowohl vom optischen Sensor 11 als auch vom Ultraschallsensor 15 ein Ereignis detektiert worden sein, wird mittels des Signalauswertemittels, das beispielsweise Bestandteil der zentralen Steuer- und/oder Regelungseinheit der Blutbehandlungsvorrichtung sein kann, erkannt, dass Luftblasen vorbeigeströmt sind. Sollte nur mittels des optischen Sensors 11 ein Ereignis detektiert worden sein, so wird mittels des Signalauswertemittels erkannt, dass ein Gerinnsel vorbeigeströmt ist.

In einem derartigen Fall werden Schutz- und Warnmittel 40 aktiviert. Im Einzelnen kann dies beispielsweise bedeuten, dass auf einem Ausgabemittel wie einem Bildschirm einer Blutbehandlungsvorrichtung, in der die erfindungsgemäße Vorrichtung verwendet wird, eine Warnmeldung ausgibt und dass weiter gegebenenfalls der extrakorporale Blutkreislauf gestoppt wird.

## Patentansprüche

1. Vorrichtung zur Ermittlung und/oder Überwachung von Fremdstrukturen (10) in Blut oder einem Blutstrom, mit wenigstens einem optischen Überwachungsmittel (11), wenigstens einem Ultraschallüberwachungsmittel (15) und wenigstens einem Signalauswertemittel, wobei das Blut oder der Blutstrom zumindest mittels des optischen Überwachungsmittels (11) optisch und zumindest mittels des Ultraschallüberwachungsmittels (15) mittels Ultraschall überwachbar ist, **dadurch gekennzeichnet, dass** anhand der Kombination der hieraus erhaltenen Überwachungssignale wenigstens eine Fremdstruktur im Blut oder Blutstrom mittels des Signalauswertemittels erkennbar und von wenigstens einer zweiten Fremdstruktur mittels des Signalauswertemittels unterscheidbar ist.

2. Vorrichtung nach Anspruch 1, **dadurch gekennzeichnet, dass** mittels des Signalauswertemittels eine oder mehrere Luftblasen im Blut oder Blutstrom erkennbar sind, wenn zumindest das Ultraschallüberwachungsmittel (15) wenigstens eine Fremdstruktur als ein signalauslösendes Ereignis registriert und ein Signal abgibt oder wenn das optische Überwachungsmittel (11) und das Ultraschallüberwachungsmittel (15) jeweils wenigstens eine Fremdstruktur als ein signalauslösendes Ereignis im Wesentlichen zeitgleich registrieren und ein Signal abgeben, und/oder dass mittels des Signalauswertemittels ein oder mehrere Festkörper im Blut oder Blutstrom erkennbar sind, wenn das optische Überwachungsmittel (11) wenigstens eine Fremdstruktur als signalauslösendes Ereignis registriert und das Ultraschallüberwachungsmittel (15) im Wesentlichen zeitgleich keine oder eine sehr kleine Fremdstruktur als signalauslösendes Ereignis registriert.

3. Vorrichtung nach Anspruch 1 oder 2, **dadurch gekennzeichnet, dass** das optische Überwachungsmittel (11) wenigstens ein optischer Sensor ist oder wenigstens einen optischen Sensor umfasst und/oder dass das Ultraschallüberwachungsmittel (15) wenigstens ein Ultraschallsensor ist oder wenigstens einen Ultraschallsensor umfasst und/oder dass mittels des optischen Überwachungsmittels (11) Luftblasen und Festkörper detektierbar und mittels des Ultraschallüberwachungsmittels (15) Luftblasen detektierbar sind.

4. Vorrichtung nach einem der vorhergehenden Ansprüche, **dadurch gekennzeichnet, dass** signalabwärts des optischen Überwachungsmittels (11) wenigstens ein Hochpassfilter angeordnet ist, mittels dessen das mittels optischer Überwachung gewonnene Überwachungssignal filterbar ist.

5. Vorrichtung nach einem der vorhergehenden Ansprüche, **dadurch gekennzeichnet, dass** die Vorrichtung eine Aufnahme aufweist, in die ein Fluidführungsmittel (30), und/oder dass die Vorrichtung einen Messkanal aufweist.

6. Vorrichtung nach einem der vorhergehenden Ansprüche, **dadurch gekennzeichnet, dass** die Vorrichtung eine begrenzte Messstrecke oder Messstelle aufweist oder ausbildet, an der sowohl das optische Überwachungsmittel (11) als auch das Ultraschallüberwachungsmittel (15) angeordnet sind.

7. Vorrichtung nach Anspruch 6, **dadurch gekennzeichnet, dass** die Messstrecke oder Messstelle in und/oder an der Aufnahme angeordnet ist und/oder dass die Messstrecke oder Messstelle von der Lichtquelle (12), dem Ultraschallsensor (15) und dem Photodetektor (13) von drei Seiten umschlossen ist.

8. Vorrichtung nach einem der vorhergehenden Ansprüche, **dadurch gekennzeichnet, dass** die Vorrichtung wenigstens ein Schutzmittel und/oder wenigstens ein Warnmittel aufweist und/oder mit wenigstens einem Schutzmittel und/oder wenigstens einem Warnmittel in Verbindung steht, wobei mittels des Schutzmittels der Blutstrom stoppbar ist und/oder wobei mittels des Warnmittels auf im Blut oder Blutstrom erkannte Fremdstrukturen hinweisbar ist.

9. Vorrichtung nach einem der vorhergehenden Ansprüche, **dadurch gekennzeichnet, dass** die Vorrichtung Bestandteil einer Blutbehandlungsvorrichtung ist oder eine Blutbehandlungsvorrichtung ist.

10. Verfahren zur Ermittlung und/oder Überwachung von Fremdstrukturen in Blut oder einem Blutstrom, wobei das Blut oder der Blutstrom optisch und mittels Ultraschall überwacht wird, **dadurch gekennzeichnet, dass** anhand der Kombination der hieraus erhaltenen Überwachungssignale wenigstens eine Fremdstruktur im Blut oder einem Blutstrom erkennbar und von wenigstens einer zweiten Fremdstruktur unterscheidbar ist.

11. Verfahren nach Anspruch 10, **dadurch gekennzeichnet, dass** eine oder mehrere Luftblasen im Blut oder Blutstrom erkannt werden, wenn zumindest mittels Ultraschallüberwachung wenigstens eine Fremdstruktur im Blut oder Blutstrom erkannt wird oder wenn sowohl mittels optischer Überwachung und Ultraschallüberwachung im Wesentlichen zeitgleich wenigstens eine Fremdstruktur im Blut oder Blutstrom erkannt wird, und/oder dass ein oder mehrere Festkörper im Blut oder Blutstrom erkannt werden, wenn optisch wenigstens eine Fremdstruktur im Blut oder Blutstrom erkannt wird und mittels Ultraschall keine oder eine sehr kleine Fremdstruktur im Blut oder Blutstrom erkannt wird.

12. Verfahren nach Anspruch 10 oder 11, **dadurch gekennzeichnet, dass** das mittels optischer Überwachung gewonnene Überwachungssignal mittels eines Hochpassfilters gefiltert wird.

13. Verfahren nach Anspruch 12, **dadurch gekennzeichnet, dass** der Hochpassfilter ein Filter ist, in dem das Überwachungssignal in festen Abständen von wenigen Millisekunden integriert und aus einer vorbestimmten Anzahl von vorangegangenen Messungen ein gleitender Mittelwert bestimmt und vom aktuellen Integrationsergebnis subtrahiert wird.

14. Verfahren nach einem der Ansprüche 10 bis 13, **dadurch gekennzeichnet, dass** das Verfahren mit wenigstens einer Vorrichtung nach einem der Ansprüche 1 bis 9 durchgeführt wird.

15. Verwendung einer Vorrichtung nach einem der Ansprüche 1 bis 9 zur Durchführung des Verfahrens gemäß Anspruch 10 bis 14 in einer Blutbehandlungsvorrichtung.

16. Blutbehandlungsvorrichtung mit wenigstens einer Vorrichtung nach einem der Ansprüche 1 bis 9.

17. Kombination eines Disposables mit einer Vorrichtung nach Anspruch 1.

## Claims

1. An apparatus for determining and/or monitoring foreign structures (10) in blood or in a bloodstream, comprising at least one optical monitoring means (11), at least one ultrasound monitoring means (15) and at least one signal evaluation means, wherein the blood or the bloodstream can be optically monitored at least by means of the optical monitoring means (11) and by means of ultrasound at least by means of the ultrasound monitoring means (15), **characterized in that** at least one foreign structure can be recognized in the blood or in the bloodstream by means of the signal evaluation means with reference to the combination of the monitoring signals obtained herefrom and can be distinguished from at least one second foreign structure by means of the signal evaluation means.

2. An apparatus in accordance with claim 1, **characterized in that** one or more air bubbles in the blood or bloodstream can be recognized by means of the signal evaluation means when at least the ultrasound monitoring means (15) registers at least one foreign structure as a signal-triggering event and outputs a signal or when the optical monitoring means (11) and the ultrasound monitoring means (15) each substantially simultaneously register at least one foreign structure as a signal-triggering event and output a signal, and/or **in that** one or more solid bodies can be recognized in the blood or in the bloodstream by means of the signal evaluation means when the optical monitoring means (11) registers at least one foreign structure as a signal-triggering event and the ultrasound monitoring means (15) substantially simultaneously does not register any foreign structure, or recognizes a very small foreign structure, as a signal-triggering event.

3. An apparatus in accordance with either of claims 1 or 2, **characterized in that** the optical monitoring means (11) is at least one optical sensor or includes at least one optical sensor; and/or **in that** the ultrasound monitoring means (15) is at least one ultrasound sensor or includes at least one ultrasound sensor; and/or **in that** air bubbles and solid bodies can be detected by means of the optical monitoring means (11) and air bubbles can be detected by means of the ultrasound monitoring means (15).

4. An apparatus in accordance with one of the preceding claims, **characterized in that** down-signal of the optical monitoring means (11) at least one high pass filter is arranged by means of which the monitoring signal acquired by means of optical monitoring can be filtered.

5. An apparatus in accordance with one of the preceding claims, **characterized in that** the apparatus has a recipient into which a fluid guidance mean (30); and/or **in that** the apparatus has a measurement channel.

6. An apparatus in accordance with one of the preceding claims, **characterized in that** the apparatus has or forms a limited measurement path or measurement point at which both the optical monitoring means (11) and the ultrasound monitoring means (15) are arranged.

7. An apparatus in accordance with claim 6, **characterized in that** the measurement path or the measurement point is arranged in and/or at the recipient; and/or **in that** the measurement path or the measurement point is surrounded by the light source (12), by the ultrasound sensor (15) and by the photodetector (13) on three sides.

8. An apparatus in accordance with one of the preceding claims, **characterized in that** the apparatus has at least one protection means and/or at least one warning means and/or is in communication with at least one protection means and/or at least one warning means, wherein the bloodstream can be stopped by means of the protection means and/or wherein attention can be drawn to foreign structures recognized in the blood or in the bloodstream by means of the warning means.

9. An apparatus in accordance with one of the preceding claims, **characterized in that** the apparatus is a component of a blood treatment apparatus or is a blood treatment apparatus.

10. A method for determining and/or monitoring foreign structures in blood or in a bloodstream, wherein the blood or the bloodstream is monitored optically and by means of ultrasound, **characterized in that** at least one foreign structure can be recognized in the blood or in a bloodstream and can be distinguished from at least one second foreign structure with reference to the combination of the monitoring signals obtained herefrom.

11. A method in accordance with claim 10, **characterized in that** one or more air bubbles are recognized in blood or in a bloodstream if at least one foreign structure is recognized in the blood or in the bloodsteam at least by ultrasound monitoring or if at least one foreign structure is recognized in the blood or in the bloodstream substantially simultaneously both by means of optical monitoring and ultrasound monitoring, and/or **in that** one or more solid bodies are recognized in the blood or in the bloodstream when at least one foreign structure is optically recognized in the blood or in the bloodstream and no foreign structure or a very small foreign structure is recognized by means of ultrasound.

12. A method in accordance with either of claims 10 or 11, **characterized in that** the monitoring signal acquired by means of optical monitoring is filtered by means of a high pass filter.

13. A method in accordance with claim 12, **characterized in that** the high pass filter is a filter in which the monitoring signal is integrated at fixed intervals of a few milliseconds and a floating mean value is determined from a predetermined number of preceding measurements and is subtracted from the then current integration result.

14. A method in accordance with one of the claims 10 to 13, **characterized in that** the method is carried out using at least one apparatus in accordance with one of the claims 1 to 9.

15. Use of an apparatus in accordance with one of the claims 1 to 9 for the carrying out of the method in accordance with claims 10 to 14 in a blood treatment apparatus.

16. A blood treatment apparatus comprising at least one apparatus in accordance with one of the claims 1 to 9.

17. A combination of a disposable with an apparatus in accordance with claim 1.

## Revendications

1. Dispositif en vue de la détection et/ou de la surveillance de structures étrangères (10) dans le sang ou un flux sanguin, avec au moins un moyen de surveillance optique (11), au moins un moyen de surveillance par ultrasons (15) et au moins un moyen d'évaluation du signal, le sang ou le flux sanguin étant surveillable au moins optiquement à l'aide du moyen de surveillance optique (11) au moins par ultrasons à l'aide du moyen de surveillance par ultrasons (15) **caractérisé en ce que**, au moyen de la combinaison des signaux de surveillance reçus à partir de ceux-ci, au moins une structure étrangère est détectable dans le sang ou le flux sanguin à l'aide du moyen d'évaluation du signal et différenciable d'au moins une seconde structure étrangère à l'aide du moyen d'évaluation du signal.

2. Dispositif selon la revendication 1, **caractérisé en ce que**, à l'aide du moyen d'évaluation du signal, une ou plusieurs bulles d'air sont détectables dans le sang ou le flux sanguin, lorsqu'au moins le moyen de surveillance par ultrasons (15) enregistre au moins une structure étrangère comme un événement déclencheur de signal et émet un signal, ou lorsque le moyen de surveillance optique (11) et le moyen de surveillance par ultrasons (15) enregistrent respectivement et principalement simultanément au moins une structure étrangère comme un événement déclencheur de signal et émettent un signal, et/ou **en ce que**, à l'aide du moyen d'évaluation du signal, un ou plusieurs corps solides sont détectables dans le sang ou le flux sanguin, lorsque le moyen de surveillance optique (11) enregistre au moins une structure étrangère comme événement déclencheur de signal et le moyen de surveillance par ultrasons (15) n'enregistre respectivement et principalement simultanément aucun ou qu'une très petite structure étrangère comme événement déclencheur de signal.

3. Dispositif selon la revendication 1 ou 2, **caractérisé en ce que** le moyen de surveillance optique (11) est au moins un capteur optique ou comprend au moins un capteur optique et/ou **en ce que** le moyen de surveillance par ultrasons (15) est au moins un capteur à ultrasons ou comprend au moins un capteur à ultrasons et/ou **en ce que**, à l'aide du moyen de surveillance optique (11), des bulles d'air et des corps solides sont détectables et à l'aide du moyen de surveillance par ultrasons (15), des bulles d'air sont détectables.

4. Dispositif selon une quelconque des revendications précédentes, **caractérisé en ce que**, en aval du signal du moyen de surveillance optique (11), au moins un filtre passe-haut est disposé, à l'aide duquel le signal de surveillance obtenu au moyen de la surveillance optique est filtrable.

5. Dispositif selon une quelconque des revendications précédentes, **caractérisé en ce que** le dispositif présente un réceptacle dans lequel un moyen de guidage du fluide (30) se trouve, et/ou **en ce que** le dispositif présente un canal de mesure.

6. Dispositif selon une quelconque des revendications précédentes, **caractérisé en ce que** le dispositif présente ou constitue un parcours de mesure ou une zone de mesure limités, sur lesquels sont disposés aussi bien le moyen de surveillance optique (11) que le moyen de surveillance par ultrasons (15).

7. Dispositif selon la revendication 6, **caractérisé en ce que** le parcours de mesure ou la zone de mesure est disposé dans et/ou sur le réceptacle et/ou en ce que le parcours de mesure ou la zone de mesure est entouré par la source lumineuse (12), le capteur à ultrasons (15) et le détecteur photos (13) par trois côtés.

8. Dispositif selon une quelconque des revendications précédentes, **caractérisé en ce que** le dispositif présente au moins un moyen de protection et/ou au moins un moyen d'avertissement et/ou se trouve en communication avec au moins un moyen de protection et/ou au moins un moyen d'avertissement, le flux sanguin étant arrêtable à l'aide du moyen de protection et/ou des structures étrangères détectées dans le sang ou le flux sanguin étant aptes au référencement à l'aide du moyen d'avertissement.

9. Dispositif selon une quelconque des revendications précédentes, **caractérisé en ce que** le dispositif forme partie intégrante d'un dispositif de traitement sanguin ou est un dispositif de traitement sanguin.

10. Procédé en vue de la détection et/ou de la surveillance de structures étrangères dans le sang ou un flux sanguin, le sang ou le flux sanguin étant surveillé optiquement et par ultrasons, **caractérisé en ce que**, au moyen de la combinaison des signaux de surveillance reçus à partir de ceux-ci, au moins une structure étrangère est détectable dans le sang ou le flux sanguin et différenciable d'au moins une seconde structure étrangère.

11. Procédé selon la revendication 10, **caractérisé en ce que**, une ou plusieurs bulles d'air sont détectées dans le sang ou le flux sanguin, lorsqu'au moins une structure étrangère est détectée dans le sang ou le flux sanguin au moins à l'aide de la surveillance par ultrasons ou lorsqu'au moins une structure étrangère est détectée dans le sang ou le flux sanguin principalement simultanément aussi bien à l'aide de la surveillance optique que de la surveillance par ultrasons, et/ou **en ce qu'**au moins un ou plusieurs corps solides sont détectés dans le sang ou le flux sanguin, lorsqu'au moins une structure étrangère est détectée optiquement dans le sang ou le flux sanguin et que, par ultrasons, aucune ou une très petite structure étrangère n'est détectée dans le sang ou le flux sanguin.

12. Procédé selon la revendication 10 ou la revendication 11, **caractérisé en ce que** le signal de surveillance obtenu à l'aide de la surveillance optique est filtré à l'aide d'un filtre passe-haut.

13. Procédé selon la revendication 12, **caractérisé en ce que** le filtre passe-haut est un filtre dans lequel le signal de surveillance est intégré à intervalles fixes de quelques millisecondes et **en ce que**, à partir d'un nombre prédéfini de mesures précédentes, une valeur moyenne mobile est déterminée et soustraite du résultat d'intégration actuel.

14. Procédé selon une quelconque des revendications 10 à 13, **caractérisé en ce que** le procédé est exécuté avec au moins un dispositif selon une quelconque des revendications 1 à 9.

15. Utilisation d'un dispositif selon une quelconque des revendications 1 à 9 en vue de l'exécution du procédé selon les revendications 10 à 14 dans un dispositif de traitement sanguin.

16. Dispositif de traitement sanguin avec au moins un dispositif selon une quelconque des revendications 1 à 9.

17. Combinaison d'un élément jetable avec un dispositif selon la revendication 1.
